# FASCICULE DE BREVET EUROPEEN

(11) **EP 1 761 236 B1**
(45) Date de publication et mention de la délivrance du brevet: **11.03.2009**
(21) Numéro de dépôt: 05752807.7
(22) Date de dépôt: 16.06.2005
(51) Int. Cl.: A61K 8/18, A23K 1/18, A61K 33/42, A23K 1/175

(54) **PROCEDE POUR INHIBER L'EFFET PREBIOTIQUE DES PROTEINES ALIMENTAIRES**
VERFAHREN ZUR HEMMUNG DER PRÄBIOTISCHEN WIRKUNG VON NAHRUNGSMITTELPROTEINEN
METHOD FOR INHIBITING PREBIOTIC EFFECT OF FOOD PROTEINS

(30) Priorité: 30.06.2004 FR 0407216
(43) Date de publication de la demande: 14.03.2007
(73) Titulaire: Royal Canin S.A., 30470 Aimargues (FR)
(72) Inventeur: NGUYEN, Tan Hung, F-56890 Saint Ave (FR); SERGHERAERT, Renaud, F-34690 Fabregues (FR)
(74) Mandataire: Tetaz, Franck Claude Edouard
(86) Numéro de dépôt international: PCT/EP2005/052804
(87) Numéro de publication internationale: WO 2006/010672

(56) Documents cités:
- WO-A-01/97821
- WO-A-93/25087
- GB-A- 1 143 399
- US-A- 4 027 043
- US-A- 4 041 149
- US-A- 4 370 314
- FAMULARO G ET AL: "TRADITIONAL AND HIGH POTENCY PROBIOTIC PREPARATIONS FOR ORAL BACTERIOTHERAPY" BIODRUGS, AUCKLAND, NZ, vol. 12, no. 6, 1999, pages 455-470, XP009001219 ISSN: 1173-8804

## Description

L'invention concerne un procédé pour inhiber l'effet probiotique ou prébiotique des protéines alimentaires vis-à-vis de la microflore bactérienne de la cavité buccale des carnivores domestiques. L'invention consiste à inhiber cet effet par l'emploi de phosphates alimentaires hydrosolubles.

La cavité buccale des chiens et des chats abrite une microflore bactérienne variée, se classant en microflore aérobie et microflore anaérobie. On trouve cette microflore sur les muqueuses buccales, sur les dents et dans la salive, cette dernière, par son état aqueux, étant le milieu de développement et aussi le véhicule de diffusion de la microflore.

A la naissance, la cavité buccale de l'animal est stérile, mais elle est vite colonisée par les bactéries aérobies et anaérobies dès que le jeune animal absorbe des aliments. Non seulement ces aliments ne sont pas stériles, mais leurs protéines, diffusées dans la salive, ou restées résiduelles sur les muqueuses, sur et entre les dents, favorisent le développement de la microflore microbienne. On dit alors que ces protéines ont un effet prébiotique ou probiotique vis-à-vis de la microflore (de *pro,* pour, et *bios,* vie, contrairement à *antibiotique*). Il est à noter que la salive, qui est produite stérilement par les glandes salivaires, n'aurait jamais pu être un milieu de développement et de diffusion de la microflore bactérienne sans la présence des protéines alimentaires.

On emploie selon la présente invention indifféremment les termes « prébiotiques » ou « probiotiques » pour définir le même effet de favoriser la croissance et/ou l'activité métabolique de microorganismes.

Malheureusement, chez les chiens et les chats, l'hygiène buccale est difficilement réalisable quotidiennement. Le rinçage de la bouche avec des désinfectants, le curetage et le brossage des dents avec du dentifrice après les repas, ne sont pas des pratiques courantes comme chez l'homme. D'un autre côté, les chiens et les chats sont de mieux en mieux nourris, souvent plusieurs fois par jour, soit avec des rations « ménagères », soit avec des aliments du commerce qu'on appelle des « petfoods ». Ces derniers peuvent être des aliments secs, humides ou semi humides, des snacks ou des friandises. Quelles que soient leur origine ou leur présentation, tous ces aliments apportent des protéines d'origine animale ou végétale, nécessaires certes à la nutrition des animaux, mais qui laissent des résidus favorables au développement de la microflore bactérienne buccale.

Le développement usuel de la flore microbienne peut conduire à des effets esthétiques indésirables, comme une mauvaise haleine transitoire qu'il convient de contrôler.

Quand cette microflore bactérienne se développe de façon excessive dans la cavité buccale, l'animal hôte peut présenter de nombreux troubles bien connus des éleveurs et des vétérinaires, d'autant que les chiens et les chats ont des replis sur leurs gencives formant des « poches gingivales » :
- halitose (mauvaise haleine),
- gingivite (inflammation de la gencive),
- parodontite ou maladie périodontale (inflammation du parodonte, c'est-à-dire de l'ensemble des tissus de support et d'attache des dents),
- pharyngite (inflammation de la muqueuse du pharynx)
- etc...

Ce développement excessif, pathologique, est associé à un trouble dans le contrôle de la flore microbienne qui se distingue du développement usuel qui provoque de simples effets esthétiques indésirables.

On peut traiter ces troubles par l'emploi d'agents antimicrobiens (Trevor Chin Quee, Trianthi Roussou and E.C.S. Chan, "In vitro activity of Rodogyl against putative periodontopathic bacteria", Antimicrobial Agents and Chemotherapy, Vol. 24, N° 3, 1983, pp. 445-447 ; K.S. Kornman, B. Siegrist, W.A. Soskolne and K. Nuki, "The predominant cultivable subgingival flora of beagle dogs following ligature placement and metronidazole therapy", Journal of Periodontal Research, Vol. 16, 1981, pp. 251-258).

Néanmoins, ces traitements avec des agents antimicrobiens sont souvent tardifs, car on ne leur fait appel que quand les troubles sont déjà bien visibles. Il est donc essentiel de trouver des moyens pour diminuer le développement excessif de la microflore bactérienne de la cavité buccale des chiens et des chats, avant qu'elle ne provoque des troubles pathologiques.

La demanderesse a découvert de façon inattendue que des phosphates alimentaires sont capables d'inhiber l'effet prébiotique des protéines alimentaires vis-à-vis de la microflore de la cavité buccale des carnivores domestiques. Il est essentiel que ces phosphates soient hydrosolubles pour être actifs dans la salive des animaux. Il est donc préférable d'employer des pyrophosphates ou des polyphosphates de sodium. L'apport de phosphate peut se faire seul, ou à travers un aliment, ou par toute préparation vétérinaire ou non. Dans tous les cas, l'homme du métier apportera le phosphate à utiliser en quantité suffisante pour qu'il se retrouve au moins au taux de 0.50% de la salive.

Selon le brevet WO 93/25087 de l'Université de l'Indiana, les phosphates, et particulièrement l'hexamétaphosphate de sodium, ont déjà été employés comme agents séquestrants et dissolvants pour empêcher la formation de cristaux de calcium qui constituent le tartre dentaire des animaux domestiques. Cependant, cet art antérieur n'a nullement décrit l'effet inhibiteur des phosphates vis-à-vis de l'action prébiotique des protéines alimentaires à l'égard de la microflore bactérienne de la cavité buccale.

La présente invention concerne donc un procédé d'inhibition de l'effet prébiotique des protéines alimentaires vis-à-vis de la microflore bactérienne buccale des animaux carnivores domestiques, ledit procédé consistant à administrer à l'animal carnivore domestique un inhibiteur d'effet prébiotique, l'inhibiteur comprenant un phosphate alimentaire hydrosoluble, choisi parmi le pyrophosphate de soduim et le tripolyphosphate de sodium.

Ce procédé d'inhibition est un procédé non thérapeutique lorsqu'il s'agit de contrôler le développement usuel de la flore bactérienne.

Ce procédé d'inhibition peut avoir une finalité thérapeutique lorsqu'il s'agit de contrôler le développement excessif de la flore bactérienne.

Selon la présente invention, l'inhibiteur peut être constitué par un seul phosphate alimentaire hydrosoluble ou par un mélange de phosphates alimentaires hydrosolubles.

Les phosphates alimentaires hydrosolubles sont bien connus de l'homme du métier, particulièrement ceux autorisés par la Directive 70/524/CEE, publiée au Journal Officiel de l'Union Européenne du 25.2.2004.

De manière préférentielle, le phosphate hydrosoluble est différent d'un hexamétaphosphate de sodium, avantageusement choisi parmi les pyrophosphates et les polyphosphates.

Selon un mode préférentiel de réalisation de l'invention, le phosphate alimentaire est employé en quantité telle qu'il se retrouve dissous à au moins 0,5% dans la salive des animaux.

Selon l'invention, le phosphate alimentaire peut être administré seul aux animaux ou être apporté en mélange avec des aliments pour animaux carnivores domestiques.

Les aliments sont choisis parmi les rations « ménagères », ou les aliments industriels secs, humides, semi-humides, snacks ou friandises.

De manière avantageuse, la quantité de phosphate alimentaire dans l'aliment complémenté est supérieure ou égale à 1 % en poids, de préférence compris entre et 2 % en poids.

Selon l'invention, l'inhibiteur peut être ajouté aux aliments de manière extemporanée ou encore préalablement mélangé.

Selon un autre mode de réalisation de l'invention, l'inhibiteur de l'effet prébiotique est administré aux animaux carnivores domestiques dans une préparation vétérinaire ou non.

Les exemples non exhaustifs et non limitatifs ci-après permettent d'illustrer l'invention.

### EXEMPLES

Pour toutes les expériences, la microflore bactérienne de la cavité buccale a été prélevée, conservée dans un milieu de conservation, puis cultivée dans de la salive artificielle selon le protocole suivant :

### Prélèvefnent de la microflores bactérienne et préparation de l'inoculum.

Deux chats mâles de race « européenne », pesant environ 5.50 kg, ont été anesthésiés avec 0.3 ml de médétomidine en solution à 0.085 g/100ml (Domitor, ND) et 0.26 ml de kétamine en solution à 10 g/1000 ml (Imalgène 1000, ND).

Chez chaque animal ainsi immobilisé, on aspire stérilement la salive avec une pipette et on gratte la base des dents, les gencives, et les poches gingivales avec le dos d'un scalpel stérile.

L'ensemble des prélèvements est transféré et bien dilué dans 100 ml d'un milieu de conservation stérile (milieu au thiocolate à la résazurine de Biokar additionné de 25% de glycérol). Le milieu de conservation contenant les prélèvements est transféré dans des tubes à microbilles (Cryobilles, ND de AES). Ces tubes sont ensuite incubés pendant 6 heures à l'étuve à 37°C, en jarre sous CO₂, avant d'être congelés pour servir ultérieurement.

Lors de l'utilisation ultérieure, chaque tube est décongelé à la température ambiante, puis mis à incuber pendant 12 heures à 37°C, en jarre sous CO₂. On dénombre les bactéries aérobies et anaérobies selon les méthodes décrites ci-après. On dilue ensuite le contenu de chaque tube avec le milieu de conservation stérile pour avoir un inoculum de 5000 (3.70 Log10) germes revivifiables dans 0,2 ml.

### Méthodes de dénombrement des bactéries.

La microflore aérobie est cultivée puis dénombrée sur milieu trypticase soja (Biokar) incubé à 37°C pendant 48 heures.

La microflore anaérobie est cultivée puis dénombrée sur milieu de Schaedler (Biokar) additionné de 5% de sang défibriné stérile de mouton, incubé à 37°C, sous CO₂ en jarre, pendant 48 heures.

### Solive artificielle.

La salive artificielle de base est préparée selon le tableau 31 de la page 244 de l'ouvrage Biological Hanbooks-Metablism, compiled and edited by Philip L. Altman and Dorothy S. Dittmer, published by Federation of American Societies for Experimental Biology, 1968.

Cette salive de base est additionnée de L-cystéine à raison de 0.5g/litre pour abaisser son potentiel redox afin de pouvoir y faire pousser à la fois la microflore bactérienne aérobie et la microflore bactérienne anaérobie. L'ensemble sera appelé par la suite « salive artificielle ».

### Expérimentation.

On répartit la salive artificielle dans des tubes à essai a raison de 20 ml par tube. Chaque tube est additionné ou non de la protéine, en présence ou non du phosphate à tester. Chaque traitement est composé de deux tubes.

L'ensemble est autoclave à 110°C pendant 15 minutes.

Chaque tube est ensuite ensemencé avec 0.2 ml d'inoculum décrit précédemment. L'ensemble est incubé à l'étuve à 37°C, sans agitation.

Au bout de 24, ou 48 ou 72 heures d'incubation, on dénombre la flore aérobie et la flore anaérobie selon les méthodes décrites précédemment.

Le résultat de chaque traitement est la moyenne des dénombrements de deux tubes d'essai exprimée en Log10 des C.F.U. (Colony Forming Units) par ml de salive artificielle.

### Expérience 1

Les traitements ont été les incorporations d'une farine de viande de volaille déshydratée (protéine DSH, de la Société des Protéines Industrielles, 56230 Berric, France, titrant 70 % de matières azotées totales) à raison respectivement de 0, 0.5, 1.0 et 1.5% dans la salive artificielle.

Le Tableau 1 montre que, en l'absence de la protéine, la microflore bactérienne aérobie et anaérobie ne pousse pas, ou très difficilement dans la salive artificielle seule. Mais dès que la protéine est présente, même au taux aussi bas que 0.5%, la microflore tant aérobie qu'anaérobie « explose » dès 24 heures d'incubation.

Cette expérience montre clairement l'effet prébiotique d'une cette protéine alimentaire vis-à-vis de la microflore bactérienne buccale.

### Expérience 2

Dans cette expérience, on teste l'inhibition de l'effet prébiotique d'un hydrolysat séché de protéine de volaille (protéine MP9007 de la Société des Protéines Industrielles titrant 72,5 % de matières azotées totales), incorporé au taux de 1% dans la salive artificielle, en présence du phosphate trisodique aux taux de 5 ou 10%.

Le Tableau 2 montre que l'effet prébiotique de la protéine MP9007 est complètement inhibé par le phosphate trisodique incorporé à 10%, tant pour la microflore aérobie que pour la microflore anaérobie.

L'effet inhibiteur du phosphate trisodique à 5%, bien que pas total, est aussi très important dès 24 heures d'incubation.

### Expérience 3

Dans cette expérience, on recommence à tester le phosphate trisodique, mais incorporé seulement à 0.5%, vis-à-vis de l'effet prébiotique de la protéine MP9007 incorporée au taux de 1% dans la salive artificielle. Fort de l'expérience précédente, on arrête l'essai après 24 heures d'incubation.

Le Tableau 3 montre que le phosphate trisodique incorporé à 0.5% diminue encore l'effet prébiotique de la protéine MP9007, tant vis-à-vis de la microflore aérobie (7.75 versus 8.16 Log10) que de la microflore anaérobie (7.75 versus 8.54 Log10).

### Expérience 4

L'essai consiste à tester l'effet inhibiteur du tripolyphosphate de sodium incorporé à 0, 0.5, 1, 1.5 et 2% vis-à-vis de l'effet prébiotique de la protéine MP9007 incorporée à 1% dans la salive artificielle.

Le Tableau 4 montre que le tripolyphosphate de sodium inhibe de façon importante l'effet prébiotique de la protéine.

### Expérience 5

Dans cet essai, on teste l'effet inhibiteur du tripolyphosphate de sodium incorporé à 0, 0.5, 1, 1.5 et 2% vis-à-vis de l'effet prébiotique d'un hydrolysat de soja déshydraté (Nurish 1500 IP, ND de Solea Company, titrant 83 % de matières azotées totales) incorporé à 1% dans la salive artificielle.

Le Tableau 5 montre que le tripolyphosphate de sodium, quel que soit son taux d'incorporation, inhibe pratiquement tout l'effet prébiotique de la protéine végétale utilisée.

### Expérience 6

Dans cet essai, on a testé l'effet inhibiteur du tripolyphosphate de sodium incorporé à 0, 0.5, 1, 1.5 et 2% vis-à-vis de l'effet prébiotique d'un hydrolysat de soja déshydraté (Nurish 1500 IP, ND de Solea Company, titrant 83 % de matières azotées totales) incorporé à 0.5 et à 1% dans la salive artificielle.

On a utilisé dans cet essai des microflores buccales prélevées chez un *chien.*

Les résultats rapportés dans le tableau 6 montrent que le tripolyphosphate de sodium inhibe l'effet prébiotique de l'hydrolysat de soja vis-à-vis des microflores buccales aérobies et anaérobies de chien. L'effet inhibiteur est particulièrement important aux taux d'incorporation de tripolyphosphate égal ou supérieurs à 1%.

**Tableau 1**

| EFFET PRÉBIOTIQUE DE LA PROTEINE DSH SUR LA MICROFLORE BUCCALE | | | | | |
|---|---|---|---|---|---|
| (C.F.U./ml, moyenne en Log10 de deux tubes par traitement) | | | | | |
| **Protéine DSH** | **Microflore** | **0 heure** | **24 heures** | **48 heures** | **72 heures** |
| 0% | Aérobie | 3.70 | < 3.00 | < 3.00 | 4.75 |
| | Anaérobie | 3.70 | < 3.00 | < 3.00 | < 3.00 |
| 0.5% | Aérobie | 3.70 | 7.85 | 8.99 | 8.01 |
| | Anaérobie | 3.70 | 7.27 | 7.84 | 7.13 |
| 1.0% | Aérobie | 3.70 | 8.55 | 10.01 | 7.98 |
| | Anaérobie | 3.70 | 8.32 | 9.97 | 7.97 |
| 1.5% | Aérobie | 3.70 | 8.19 | 10.28 | 8.19 |
| | Anaérobie | 3.70 | 8.27 | 9.48 | 7.50 |

**Tableau 2**

| INHIBITION DE L'EFFET PRÉBIOTIQUE DE LA PROTEINE MP9007 PAR LE PYROPHOSPHATE TRISODIQUE A 5 ET 10% | | | | | | |
|---|---|---|---|---|---|---|
| (C.F.U./ml, moyenne en Log10 de deux tubes par traitement) | | | | | | |
| **Protéine MP9007** | **Pyrophosphate trisodique** | **Microflore** | **0 heure** | **24 heures** | **48 heures** | **72 heures** |
| 0% | 0% | Aérobie 3.70 | | < 4.00 | < 4.00 | < 4.00 |
| | | Anaérobie | 3.70 | < 4.00 | < 4.00 | < 4.00 |
| 1% | 0% | Aérobie | 3.70 | 8.79 | 7.51 | 8.05 |
| | | Anaérobie | 3.70 | 8.43 | 7.72 | 7.59 |
| 1% | 5% | Aérobie | 3.70 | 6.88 | 7.56 | 7.69 |
| | | Anaérobie | 3.70 | 6.88 | 7.46 | 6.26 |
| 1% | 10% | Aérobie | 3.70 | < 4.00 | 4.88 | < 4.00 |
| | | Anaérobie | 3.70 | < 4.00 | < 4.00 | < 4.00 |

**Tableau 3**

| INHIBITION DE L'EFFET PRÉBIOTIQUE DE LA PROTEINE MP9007 PAR LE PYROPHOSPHATE TRISODIQUE A 0.5% | | | | |
|---|---|---|---|---|
| (C.F.U./ml, moyenne en Log10 de deux tubes par traitement) | | | | |
| **Protéine MP9007** | **Pyrophosphate trisodique** | **Microflore** | **0 heure** | **24 heures** |
| 0% | 0% | Aérobie | 3.70 | < 3.00 |
| | | Anaérobie | 3.70 | < 3.00 |
| 1% | 0% | Aérobie | 3.70 | 8.16 |
| | | Anaérobie | 3.70 | 8.54 |
| 1% | 0.5% | Aérobie | 3.70 | 7.75 |
| | | Anaérobie | 3.70 | 7.75 |

**Tableau 4**

| INHIBITION DE L'EFFET PRÉBIOTIQUE DE LA PROTEINE MP9007 PAR LE TRIPOLYPHOSPHATE DE SODIUM | | | | | |
|---|---|---|---|---|---|
| (C.F.U./ml, moyenne en Log10 de deux tubes par traitement) | | | | | |
| **Protéine MP9007** | **Tripolyphosphate** | **Microflore** | **0 heure** | **24 heures** | **48 heures** |
| 1% | 0% | Aérobie | 3.70 | 7.97 | 7.69 |
| | | Anaérobie | 3.70 | 7.90 | 7.97 |
| 1% | 0.5% | Aérobie | 3.70 | 5.50 | 6.90 |
| | | Anaérobie | 3.70 | 5.41 | 7.04 |
| 1% | 1% | Aérobie | 3.70 | 4.81 | 6.49 |
| | | Anaérobie | 3.70 | < 4.00 | 6.36 |
| 1% | 1.5% | Aérobie | 3.70 | 4.84 | 6.83 |
| | | Anaérobie | 3.70 | 4.98 | 6.82 |
| 1% | 2% | Aérobie | 3.70 | < 4.00 | < 4.00 |
| | | Anaérobie | 3.70 | < 4.00 | < 4.00 |

**Tableau 5**

| INHIBITION DE L'EFFET PRÉBIOTIQUE DE L'HYDROLYSAT DE SOJA PAR LE TRIPOLYPHOSPHATE DE SODIUM | | | | | |
|---|---|---|---|---|---|
| (C.F.U./ml, moyenne en Lolo de deux tubes par traitement) | | | | | |
| **Hydrolysat de soja** | **Tripolyphosphate** | **Microflore** | **0 heure** | **24 heures** | **48 heures** |
| 1% | 0% | Aérobie | 3.70 | 6.93 | 8.31 |
| | | Anaérobie | 3.70 | 7.11 | 8.10 |
| 1% | 0.5% | Aérobie | 3.70 | < 4.00 | < 4.00 |
| | | Anaérobie | 3.70 | < 4.00 | < 4.00 |
| 1% | 1% | Aérobie | 3.70 | < 4.00 | < 4.00 |
| | | Anaérobie | 3.70 | < 4.00 | < 4.00 |
| 1% | 1.5% | Aérobie | 3.70 | < 4.00 | < 4.00 |
| | | Anaérobie | 3.70 | < 4.00 | < 4.00 |
| 1% | 2% | Aérobie | 3.70 | < 4.00 | < 4.00 |
| | | Anaérobie | 3.70 | < 4.00 | < 4.00 |

**Tableau 6**

| INHIBITION DE L'EFFET PRÉBIOTIQUE DE L'HYDROLYSAT DE SOJA VIS-A-VIS D'UNE *FLORE BUCCALE DE CHIEN* PAR LE TRIPOLYPHOSPHATE DE SODIUM | | | | | |
|---|---|---|---|---|---|
| (C.F.U./ml, moyenne en Log10 de deux tubes par traitement) | | | | | |
| **Tripolyphosphate** | **Hydrolysat de soja** | **Microflore** | **0 heure** | **24 heures** | **48 heures** |
| 0% | 0% | Aérobie | 3 | < 3 | < 3 |
| | | Anaérobie | 3 | < 3 | < 3 |
| 0% | 0.5% | Aérobie | 3 | 7.47 | 8.06 |
| | | Anaérobie | 3 | 6.67 | 6.94 |
| 0% | 1% | Aérobie | 3 | 7.94 | 8.01 |
| | | Anaérobie | 3 | 6.72 | 7.12 |
| 0.5% | 0.5% | Aérobie | 3 | 7.10 | 7.54 |
| | | Anaérobie | 3 | 1.92 | 5.80 |
| 0.5% | 1% | Aérobie | 3 | 7.50 | 7.51 |
| | | Anaérobie | 3 | 6.30 | 6.24 |
| 1% | 0.5% | Aérobie | 3 | < 3 | < 3 |
| | | Anaérobie | 3 | < 3 | < 3 |
| 1% | 1% | Aérobie | 3 | < 3 | 3.17 |
| | | Anaérobie | 3 | < 3 | 3.30 |
| 1.5% | 0.5% | Aérobie | 3 | < 3 | < 3 |
| | | Anaérobie | 3 | < 3 | < 3 |
| 1.5% | 1% | Aérobie | 3 | < 3 | < 3 |
| | | Anaérobie | 3 | < 3 | < 3 |
| 2% | 0.5% | Aérobie | 3 | < 3 | < 3 |
| | | Anaérobie | 3 | < 3 | < 3 |
| 2% | 1% | Aérobie | 3 | < 3 | < 3 |
| | | Anaérobie | 3 | < 3 | < 3 |

## Revendications

1. Utilisation d'un phosphate alimentaire hydrosoluble, choisi parmi le pyrophosphate de sodium et tripolyphosphate de sodium, pour la préparation d'un inhibiteur de l'effet prébiotique des protéines alimentaires vis-à-vis de la microflore bactérienne buccale des animaux carnivores domestiques, destiné à la prévention ou au traitement des troubles associés au développement de la microflore bactérienne buccale des animaux.

2. Utilisation selon la revendication 1, dans laquelle le phosphate alimentaire se retrouve dissous à au moins 0.5% dans la salive des animaux.

3. Utilisation selon l'une des revendications 1 à 2, dans laquelle l'inhibiteur est destiné à être administré seul aux animaux.

4. Utilisation selon l'une des revendications 1 à 3, dans laquelle l'inhibiteur est destiné à être apporté en mélange avec des aliments pour animaux carnivores domestiques.

5. Utilisation selon la revendication 4, **caractérisé en ce que** l'inhibiteur est destiné à être ajouté aux aliments de manière extemporanée.

6. Utilisation selon la revendication 5, **caractérisé en ce que** l'inhibiteur est destiné à être préalablement mélangé aux aliments.

7. Utilisation selon l'une des revendications 5 à 6, dans lequel les aliments sont choisis parmi les rations « ménagères », ou les aliments industriels secs, humides, semi-humides, snacks ou friandises.

## Claims

1. Use of a water-soluble food phosphate, chosen from sodium pyrophosphate and sodium tripolyphosphate, for the preparation of an inhibitor of the prebiotic effect of food proteins on the oral bacterial microflora of carnivorous domestic animals, for use in the prevention or treatment of conditions associated with the development of the oral bacterial microflora of animals.

2. Use according to Claim 1, in which the food phosphate is dissolved at at least 0.5% in the animals' saliva.

3. Use according to either of Claims 1 and 2, in which the inhibitor is intended to be administered on its own to the animals.

4. Use according to one of Claims 1 to 3, in which the inhibitor is intended to be provided as a mixture with foods for carnivorous domestic animals.

5. Use according to Claim 4, **characterized in that** the inhibitor is intended to be added to the foods extemporaneously.

6. Use according to Claim 5, **characterized in that** the inhibitor is intended to be premixed with the foods.

7. Use according to either of Claims 5 and 6, in which the foods are chosen from "household" rations, or dry, moist or semi-moist industrial foods, snacks or treats.

## Patentansprüche

1. Verwendung eines wasserlöslichen, für Nahrungsmittel geeigneten Phosphats, das aus Natriumpyrophosphat und Natriumtripolyphosphat gewählt ist, zur Herstellung eines Mittels, das die präbiotische Wirkung der Nahrungsproteine auf die bakterielle Mundflora fleischfressender Haustiere hemmt und zur Vorbeugung und Behandlung von Störungen, die mit der Entwicklung der bakteriellen Mundflora der Tiere in Verbindung stehen, bestimmt ist.

2. Verwendung nach Anspruch 1, wobei das für Nahrungsmittel geeignete Phosphat sich derart im Speichel der Tiere löst, dass seine Konzentration dort mindestens 0,5 % beträgt.

3. Verwendung nach einem der Ansprüche 1 bis 2, wobei das hemmend wirkende Mittel zur alleinigen Verabreichung an die Tiere bestimmt ist.

4. Verwendung nach einem der Ansprüche 1 bis 3, wobei das hemmend wirkende Mittel dazu bestimmt ist, in Mischung mit Nahrungsmitteln für fleischfressende Haustiere zugeführt zu werden.

5. Verwendung nach Anspruch 4, **dadurch gekennzeichnet, dass** das hemmend wirkende Mittel dazu bestimmt ist, den Nahrungsmitteln erst unmittelbar vor dem Verzehr zugesetzt zu werden.

6. Verwendung nach Anspruch 5, **dadurch gekennzeichnet, dass** das hemmend wirkende Mittel dazu bestimmt ist, den Nahrungsmitteln bereits im Vorfeld zugesetzt zu werden.

7. Verwendung nach einem der Ansprüche 5 bis 6, wobei die Nahrungsmittel aus im Haushalt zubereitetem Futter oder aus Trockenfutter, Nassfutter, Halbtrockenfutter, Snacks und Leckereien aus industrieller Herstellung gewählt sind.
